# EUROPEAN PATENT APPLICATION

(11) **EP 4 399 976 A1**
(43) Date of publication of application: **17.07.2024**
(21) Application number: 22864770.7
(22) Date of filing: 06.09.2022
(51) Int. Cl.: A23C 20/02, A23J 3/00

(54) **METHOD FOR MANUFACTURING CHEESE ANALOG USING ENZYME**

(30) Priority: 06.09.2021 JP 2021144901
(71) Applicant: AJINOMOTO CO., INC., Chuo-ku Tokyo 104-8315 (JP)
(72) Inventor: FUJIMURA, Yuko, Kawasaki-shi, Kanagawa 210-8681 (JP); USUGI, Koudai, Kawasaki-shi, Kanagawa 210-8681 (JP); KITAZAWA, Daisuke, Kawasaki-shi, Kanagawa 210-8681 (JP)
(74) Representative: Strehl Schübel-Hopf & Partner
(86) International application number: PCT/JP2022/033455
(87) International publication number: WO 2023/033188

(57) **Abstract**

The present invention aims to provide a method for producing cheese analogues with improved meltability and/or extensibility when heated, and a method for producing cheese analogues, in which gelation of a mixture of raw material components during production and/or separation of oil are/is suppressed.

A method for producing a cheese analogue, including a step of causing protease to act on a mixture containing fat and/or oil, starch, and protein. A method for producing a cheese analogue containing fat and/or oil, starch and protein, including (a) a step of causing protease to act on a mixture containing fat and/or oil, starch, and protein, and (b) a step of mixing with stirring the mixture obtained in step (a).

## Description

### [Technical Field]

The present invention relates to a method for producing cheese analogues with improved meltability and/or extensibility when heated, a method for improving meltability and/or extensibility of cheese analogues when heated, and an enzyme preparation used for those methods.

The present invention also relates to a method for producing cheese analogues, in which gelation of a mixture of raw material components and/or separation of oil during production are/is suppressed, a method for suppressing gelation of a mixture of raw material components and/or separation of oil during production of cheese analogues, and enzyme preparations used for those methods.

### [Background Art]

Cheese analogues (also referred to as analogue cheese, imitation cheese, etc.) are processed foods that have the appearance and texture of cheese as a substitute for cheese (for example, food products in which a part or all of the fat and protein in cheese are replaced with plant-derived components). In recent years, the market is expanding with the rise in animal welfare awareness and health consciousness.

Commercially available cheese analogues currently on the market contain starch to impart them with a smooth milk cheese-like texture, shape retention, and meltability after heating. Cheese analogues are advantageous in that they can be manufactured more easily than cheeses made using raw materials derived from milk, since they can be manufactured without undergoing fermentation or aging processes. On the other hand, because cheese analogues contain relatively large amounts of starch, which is a carbohydrate, and relatively small amounts of protein, they are not desirable for consumers who prefer nutritionally balanced food products.

From the aspect of improving nutritional balance, the present inventors attempted to manufacture conventional cheese analogues that use starch as a raw material, by blending an increased amount of protein. As a result, it was found that the obtained cheese analogues had a problem of poor meltability and poor spreadability when heated.

Therefore, the development of a cheese analogue that has good meltability and spreadability when heated, even when a large amount of protein is blended, is desired.

Methods for producing cheese analogues generally include a production method including emulsifying raw materials such as starch, fats, oils, and the like by stirring, filling same into molds, and heating same without stirring (heating for gelatinization), followed by cooling, and a production method including heating with stirring the raw material components such as starch, fats, oils, and the like (heating for gelatinization), and filling same into molds, followed by cooling. Assuming production in a factory, the latter method is preferred since cheese analogues can be produced efficiently. However, when the blending amount of protein was increased in conventional cheese analogues that use starch, fats, and oils as raw materials, a problem was found that a mixture of the raw material components became a gel by heating them while stirring, the gel was disintegrated by stirring, and separation of oil was further developed, resulting in poor moldability of the cheese analogue.

Therefore, the development of a method for suppressing the gelation of a mixture of raw material components and the separation of oil during the step of heating the raw material components while stirring, even when a large amount of protein is blended, is desired.

Patent Literature 1 discloses a food product containing a plant-derived protein that uses transglutaminase in the production step, and discloses that the food product may further contain a protease.

Patent Literature 2 discloses a fermented food product obtained by fermenting, by using moromi containing Aspergillus oryzae, a plant-derived protein gel product flocculated using an enzyme having soybean milk flocculating activity produced by Bacillus bacteria, and alkaline serine proteinase has been described as such enzyme.

Patent Literatures 1 and 2 do not describe or suggest at all the problem that when the amount of protein blended in cheese analogues that use starch, fats, and oils as raw materials is increased, the meltability and extensibility of the cheese analogues are deteriorated when heated, that gelation and oil separation occur during production of cheese analogues, or solutions to these problems.

### [Citation List]

### [Patent Literature]

[Patent Literature 1]
   US-A-2017/0150734
[Patent Literature 2]
   JP-A-2004-129523

### [Summary of Invention]

### [Technical Problem]

The present invention aims to provide a method for producing cheese analogues with improved meltability and/or extensibility when heated. The present invention also aims to provide a method for producing cheese analogues, in which gelation of a mixture of raw material components and/or separation of oil during production are/is suppressed.

### [Solution to Problem]

The present inventors have conducted intensive studies in an attempt to solve the aforementioned problems and found that the production of cheese analogues with improved meltability and/or extensibility when heated becomes possible by causing protease to act on a mixture containing fat and/or oil, starch, and protein.

In addition, the present inventors have found that, during the production of cheese analogues, gelation of a mixture containing fat and/or oil, starch, and protein and/or separation of oil can be suppressed by a step of causing protease to act on the mixture and then heating the mixture with stirring.

Based on the above-mentioned findings, the present inventor have further conducted intensive studies and completed the present invention.

That is, the present invention provides the following.
[1] A method for producing a cheese analogue, comprising a step of causing protease to act on a mixture comprising fat and/or oil, starch, and protein.
[2] The production method of the above-mentioned [1], wherein the protease is selected from the group consisting of
   (1) an endo-type/exo-type protease,
   (2) a combination of an endo-type protease and an exo-type protease,
   (3) an exo-type protease, and
   (4) an endo-type protease.
[3] The production method of the above-mentioned [1] or [2], further comprising causing α-glucosidase to act on the aforementioned mixture comprising fat and/or oil, starch, and protein.
[4] A method for improving meltability and/or extensibility of a cheese analogue when heated, comprising a step of causing protease to act on a mixture comprising fat and/or oil, starch, and protein during production of the cheese analogue.
[5] The method for improving meltability and/or extensibility of the above-mentioned [4], wherein the protease is selected from the group consisting of
   (1) an endo-type/exo-type protease,
   (2) a combination of an endo-type protease and an exo-type protease,
   (3) an exo-type protease, and
   (4) an endo-type protease.
[6] The method for improving meltability and/or extensibility of the above-mentioned [4] or [5], further comprising causing α-glucosidase to act on the aforementioned mixture comprising fat and/or oil, starch, and protein.
[7] An enzyme preparation for improving meltability and/or extensibility of a cheese analogue when heated, which preparation comprising protease, wherein the preparation is for causing the protease to act on a mixture comprising fat and/or oil, starch, and protein during production of the cheese analogue.
[8] The enzyme preparation of the above-mentioned [7], wherein the protease is selected from the group consisting of
   (1) an endo-type/exo-type protease,
   (2) a combination of an endo-type protease and an exo-type protease,
   (3) an exo-type protease, and
   (4) an endo-type protease.
[9] The enzyme preparation of the above-mentioned [7] or [8], comprising the aforementioned protease and α-glucosidase for causing the α-glucosidase to act on the aforementioned mixture comprising fat and/or oil, starch, and protein.
[10] A method for producing a cheese analogue comprising fat and/or oil, starch and protein, comprising
   (a) a step of causing protease to act on a mixture comprising fat and/or oil, starch, and protein, and
   (b) a step of heating with stirring the mixture obtained in step (a).
[11] The production method of the above-mentioned [10], wherein the protease is selected from the group consisting of
   (1) an endo-type/exo-type protease,
   (2) a combination of an endo-type protease and an exo-type protease,
   (3) an exo-type protease, and
   (4) an endo-type protease.
[12] The production method of the above-mentioned [10] or [11], wherein the aforementioned mixture further comprises a polysaccharide thickener.
[13] A method for suppressing gelation of a mixture comprising fat and/or oil, starch, and protein and/or separation of oil in step (b), comprising (a) a step of causing protease to act on the mixture, and (b) a step of heating with stirring the mixture obtained in step (a), during production of a cheese analogue comprising fat and/or oil, starch and protein.
[14] The suppressing method of the above-mentioned [13], wherein the protease is selected from the group consisting of
   (1) an endo-type/exo-type protease,
   (2) a combination of an endo-type protease and an exo-type protease,
   (3) an exo-type protease, and
   (4) an endo-type protease.
[15] The suppression method of the above-mentioned [13] or [14], wherein the aforementioned mixture further comprises a polysaccharide thickener.
[16] An enzyme preparation for suppressing gelation of the aforementioned mixture comprising fat and/or oil, starch, and protein and/or separation of oil, which preparation comprising protease, for causing the protease to act on the aforementioned mixture, in the method of any of the above-mentioned [13] to [15] .

### [Advantageous Effects of Invention]

According to the present invention, cheese analogues with improved meltability and/or extensibility when heated can be produced by causing protease to act on a mixture containing fat and/or oil, starch, and protein.

According to the present invention, cheese analogues containing a large amount of protein and having improved meltability and/or extensibility when heated can be provided.

According to the present invention, gelation of a mixture containing fat and/or oil, starch, and protein and/or separation of oil can be suppressed during production of cheese analogues, by a step of causing protease to act on the mixture and then heating the mixture with stirring.

According to the present invention, cheese analogues can be produced by an efficient production method of heating a mixture of raw material components with stirring, even when the mixture of raw material components contains a large amount of protein.

According to the production method of the present invention, the above-mentioned effects can be achieved even when an emulsifier is not used.

### [Brief Description of Drawings]

[Fig. 1]

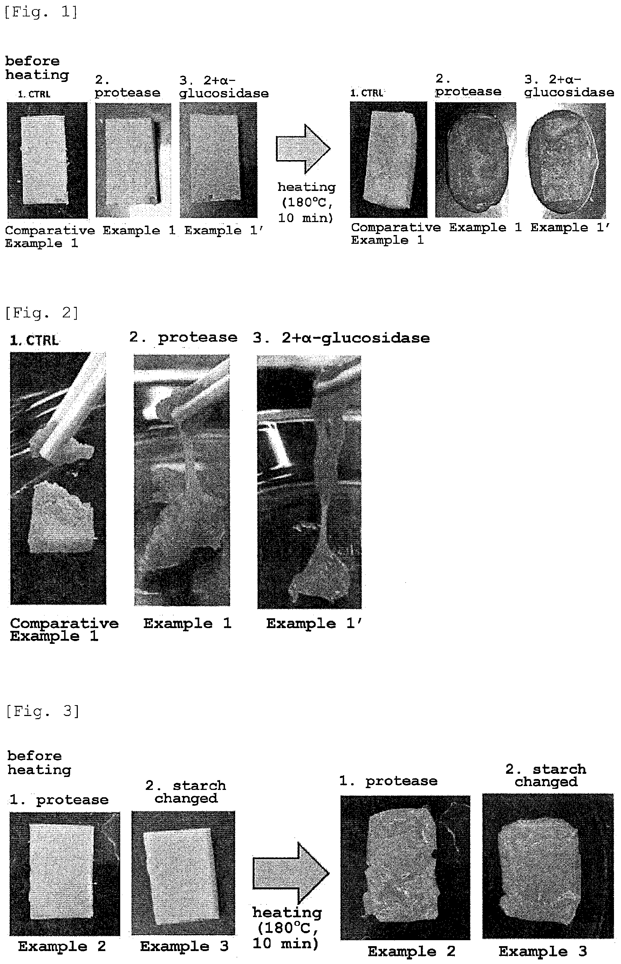

   Fig. 1 shows photographs observing the meltability of the cheese analogues of Comparative Example 1, Example 1, and Example 1' when heated in Experimental Example 1.
[Fig. 2]
   Fig. 2 shows photographs observing the extensibility of the cheese analogues of Comparative Example 1, Example 1, and Example 1' when heated in Experimental Example 1.
[Fig. 3]
   Fig. 3 shows photographs observing the meltability of the cheese analogues of Examples 2 and 3 when heated in Experimental Example 2.
[Fig. 4]

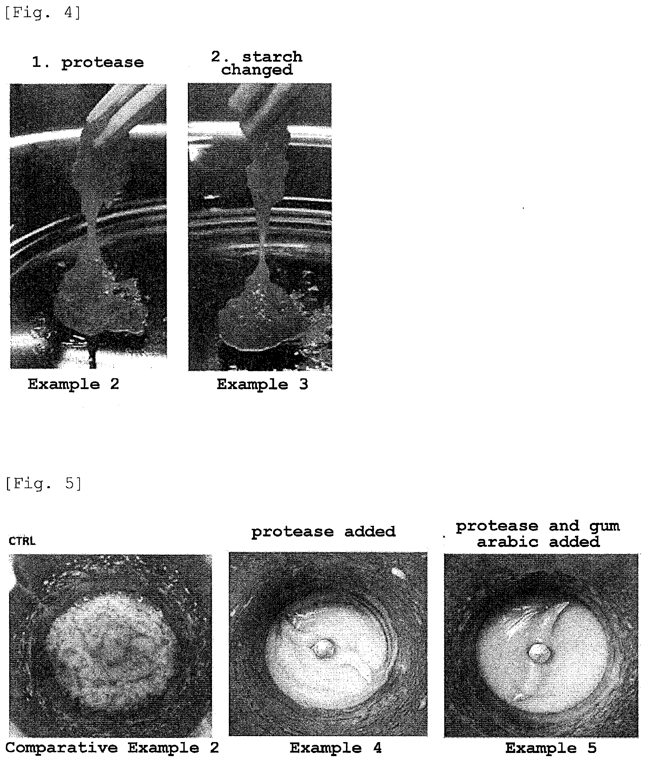

   Fig. 4 shows photographs observing the extensibility of the cheese analogues of Examples 2 and 3 when heated in Experimental Example 2.
[Fig. 5]
   Fig. 5 shows photographs of mixtures of raw material components during production of cheese analogues in Comparative Example 2 and Examples 4 and 5, in Experimental Example 3.
[Fig. 6]

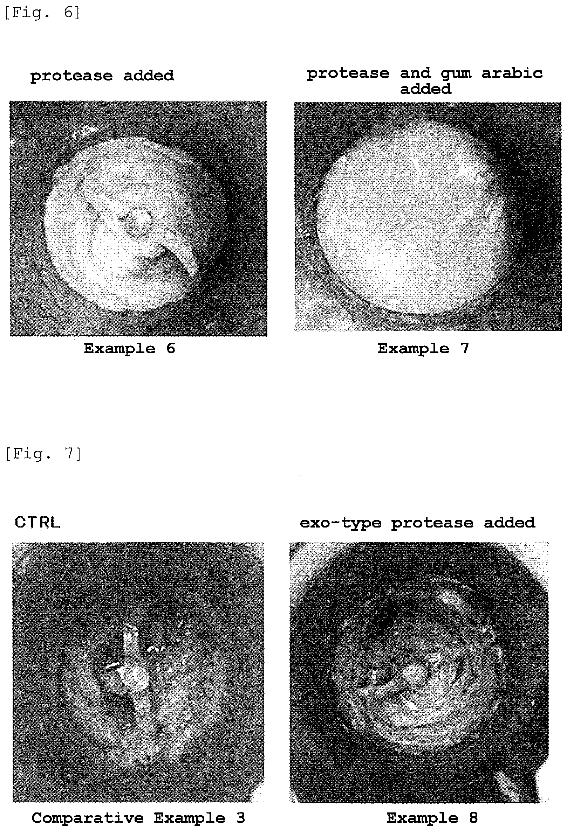

   Fig. 6 shows photographs of mixtures of raw material components during production of cheese analogues in Examples 6 and 7, in Experimental Example 4.
[Fig. 7]
   Fig. 7 shows photographs of mixtures of raw material components during production of cheese analogues in Comparative Example 3 and Example 8, in Experimental Example 5.
[Fig. 8]

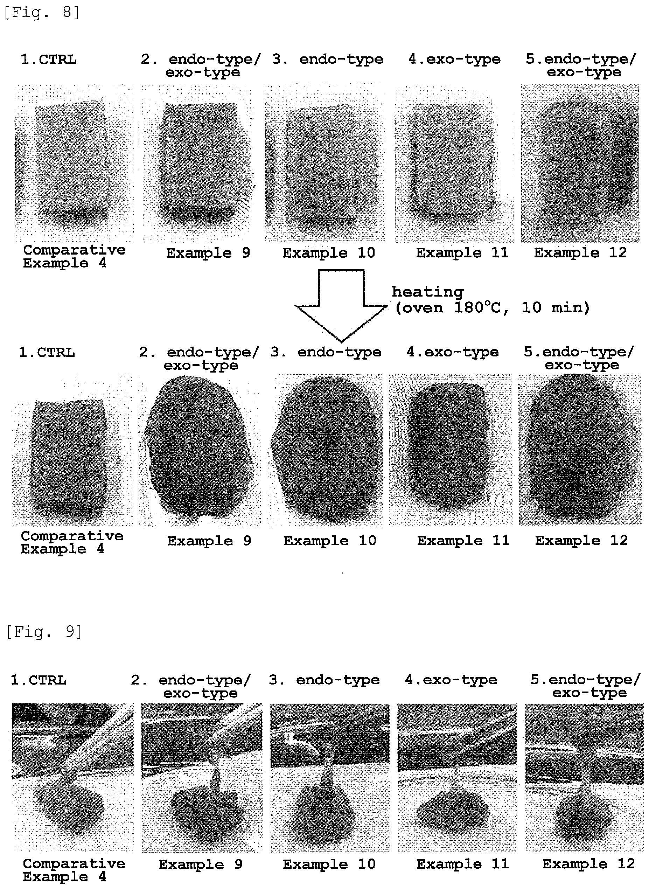

   Fig. 8 shows photographs observing the meltability of the cheese analogues of Comparative Example 4 and Examples 9 to 12 when heated in Experimental Example 6.
[Fig. 9]
   Fig. 9 shows photographs observing the extensibility of the cheese analogues of Comparative Example 4 and Examples 9 to 12 when heated in Experimental Example 6.

### [Description of Embodiments]

The present invention is described in detail below.

The method for producing cheese analogues of the present invention characteristically includes causing protease to act on a mixture containing fat and/or oil, starch, and protein.

As embodiments of the method for producing cheese analogues of the present invention, the following (I), (II) can be mentioned.
(I) A method for producing a cheese analogue, including a step of causing protease (preferably, protease and α-glucosidase) to act on a mixture containing fat and/or oil, starch, and protein (hereinafter to be also indicated as the production method (I) of the present invention).
(II) A method for producing a cheese analogue containing fat and/or oil, starch and protein, including
   (a) a step of causing protease to act on a mixture containing fat and/or oil, starch, and protein, and
   (b) a step of heating with stirring the mixture obtained in step (a)
   (hereinafter to be also indicated as the production method (II) of the present invention).

In the present specification, Unless particularly indicated, "the production method of the present invention" include the production methods (I) and (II) of the present invention.

The "fat and/or oil" used in the present invention refers to those generally used for food products.

The "fat and/or oil" in the present invention includes, for example, plant-derived fat and/or oil such as coconut oil, palm oil, rape seed oil, soybean oil, corn oil, safflower oil, cacao oil and the like; and animal-derived fat and/or oil such as beef tallow, lard, chicken fat, and the like. Plant-derived fat and/or oil are/is preferred, and coconut oil is more preferred. Fat and/or oil may be used alone or in combination of two or more.

The "starch" used in the present invention refers to plant-derived raw starch and modified starch generally used for food products.

The starch in the present invention includes, for example, rice starch, sago starch, tapioca starch, waxy corn starch, regular corn starch, potato starch, wheat starch, and dry-heat treatment starches of these plant-derived starches, and chemical treatment starches of these plant-derived starches such as hydroxypropyl distarch phosphate, acetylated distarch adipate, acetylated distarch phosphate, acetylated oxidized starch, starch sodium octenylsuccinate, starch acetate, oxidized starch, hydroxypropyl starch, phosphated distarch phosphate, monostarch phosphate, distarch phosphate. Sago starch, waxy corn starch, and tapioca starch are preferred. Starch may be used alone or in combination of two or more.

The "proteins" used in the present invention are those generally used for food products, and non-animal-derived protein (plant-derived protein, protein derived from microorganism, protein derived from fungi) is preferred.

The protein in the present invention includes, for example, plant-derived proteins such as almond protein, soybean protein, pea protein, chickpea protein, fava bean protein, oat protein, chia seed protein, rape seed protein, floating grass protein, and the like; protein derived from microorganism; protein derived from fungi, and the like, and plant-derived protein is preferred, and almond protein is more preferred. Protein may be used alone or in combination of two or more.

The protease used in the present invention is an enzyme that catalyzes the hydrolysis of peptide bonds in proteins. In the present invention, proteases having any substrate specificity and any reaction property can be used as long as they have the activity and are capable of degrading animal-derived proteins. In addition, the origin thereof is not particularly limited, and proteases of any origin can be used, such as those derived from plants (e.g., derived from papaya), those derived from mammal, those derived from fish, those derived from microorganism (e.g., derived from Aspergillus spp., derived from Bacillus spp., derived from Rhizopus spp.), and the like, and recombinant enzymes may also be used.

In the present invention, as the activity unit of endo-type protease, the amount of enzyme that causes an increase in the colored substance of Folin reagent corresponding to 1 µg of tyrosine per minute using casein as a substrate is defined as 1 unit (1 U).

In the present invention, as the activity unit of exo-type protease, the activity of producing 1 µmol of p-nitroaniline per minute using L-leucyl-p-nitroanilide as a substrate is defined as 1 unit (1 U).

In the present invention, protease includes endo-type/exo-type protease, endo-type protease, exo-type protease, and combinations thereof (e.g., combinations of endo-type protease and exo-type protease).

In the production method of the present invention, protease is preferably selected from the group consisting of (1) an endo-type/exo-type protease, (2) a combination of an endo-type protease and an exo-type protease, (3) an exo-type protease, and (4) an endo-type protease; more preferably selected from the group consisting of (1) an endo-type/exo-type protease, (2) a combination of an endo-type protease and an exo-type protease, and (4) an endo-type protease; further preferably selected from the group consisting of (1) an endo-type/exo-type protease, and (2) a combination of an endo-type protease and an exo-type protease.

The endo-type/exo-type protease used in the present invention is an enzyme that hydrolyzes peptide bonds within proteins and peptide bonds at the terminals of proteins into several peptides or amino acids.

The endo-type/exo-type protease used in the present invention may be a commercially available product and, for example, Proteax (manufactured by Amano Enzyme Inc.; derived from Aspergillus oryzae), peptidase R (manufactured by Amano Enzyme Inc.; derived from Rhizopus oryzae), Denateam AP (manufactured by Nagase ChemteX Corporation; derived from Aspergillus oryzae), purified papain for food products (manufactured by Nagase ChemteX Corporation; derived from papaya latex) can be mentioned.

The endo-type protease used in the present invention is an enzyme that hydrolyzes peptide bonds within proteins into several peptides.

The endo-type protease used in the present invention may be a commercially available product and, for example, Protin SD-NY10 (manufactured by Amano Enzyme Inc.; derived from Bacillus amyloliquefaciens), Protin SD-AY10 (manufactured by Amano Enzyme Inc.; derived from Bacillus licheniformis), Denapsin 2P (manufactured by Nagase ChemteX Corporation; derived from Aspergillus niger), and Bioprase SP-20FG (manufactured by Nagase ChemteX Corporation; derived from Bacillus licheniformis) can be mentioned.

The exo-type protease used in the present invention is an enzyme that hydrolyzes peptide bonds at the amino terminal or carboxyl terminal of proteins to liberate amino acids.

The exo-type protease used in the present invention may be a commercially available product. The exo-type protease used in the present invention may be, for example, aminopeptidase (purified product). For example, Denateam LEP 10P (manufactured by Nagase ChemteX Corporation) can be mentioned.

The α-glucosidase (EC3.2.1.20) used in the present invention is an enzyme that hydrolyzes non-reducing terminal α-1,4-glucoside bonds and generates α-glucose. Among α-glucosidases, trans glucosidase is preferred. Enzymes commercially available from Amano Enzyme Inc. under the trade names of "trans glucosidase "Amano"" and "a-glucosidase "Amano"" are some embodiments of α-glucosidase.

Regarding the enzyme activity of α-glucosidase, 1 ml of 0.02 M acetate buffer (pH 5.0) is added to 1 ml of 1 mM α-methyl-D-glucoside, 0.5 ml of enzyme solution is added, the mixture is reacted at 40°C for 60 min, and the amount of enzyme that produces 1 µg of glucose in 2.5 ml of the reaction mixture was defined as 1 U (unit).

In the production method of the present invention, the amount of fat and/or oil to be used is, for example, 2.5 wt% or more, preferably 5 wt% or more, more preferably 7.5 wt% or more, further preferably 10 wt% or more, with respect to the cheese analogue.

In the production method of the present invention, the amount of fat and/or oil to be used is, for example, 70 wt% or less, preferably 60 wt% or less, more preferably 50 wt% or less, further preferably 40 wt% or less, with respect to the cheese analogue.

In the production method of the present invention, the amount of fat and/or oil to be used is, for example, 2.5 to 70 wt%, preferably 5 to 60 wt%, more preferably 7.5 to 50 wt%, further preferably 10 to 40 wt%, with respect to the cheese analogue.

In the production method of the present invention, when the amount of fat and/or oil used is less than 2.5 wt% with respect to the cheese analogue, the analogue tends to be an unappetizing cheese analogue with a powdery texture and tends to lack melting properties after heating.

In the production method of the present invention, when the amount of fat and/or oil used exceeds 70 wt% with respect to the cheese analogue, the analogue tends to lack shape retention, resulting in an overly soft and unappetizing cheese analogue, and oil separation tends to occur during production.

In the production method of the present invention, the amount of starch to be used is, for example, 5 wt% or more, preferably 7.5 wt% or more, more preferably 10 wt% or more, further preferably 12.5 wt% or more, with respect to the cheese analogue.

In the production method of the present invention, the amount of starch to be used is, for example, 70 wt% or less, preferably 60 wt% or less, more preferably 50 wt% or less, further preferably 40 wt% or less, with respect to the cheese analogue.

In the production method of the present invention, the amount of starch to be used is, for example, 5 to 70 wt%, preferably 7.5 to 60 wt%, more preferably 10 to 50 wt%, further preferably 12.5 to 40 wt%, with respect to the cheese analogue.

In the production method of the present invention, by setting the amount of use of starch to fall within the above-mentioned ranges, the effect of imparting a milk cheese-like smooth texture and shape retention can be obtained.

In the production method of the present invention, when the amount of use of starch is less than 5 wt% with respect to the cheese analogue, the analogue tends to lack shape retention, resulting in an overly soft and unappetizing cheese analogue.

In the production method of the present invention, when the amount of use of starch exceeds 70 wt% with respect to the cheese analogue, the analogue tends to be an unappetizing cheese analogue with a powdery texture, the viscosity of the mixture increases excessively during production, making mixing difficult.

In the production method of the present invention, the amount of protein to be used is, for example, 0.1 wt% or more, preferably 0.2 wt% or more, more preferably 0.5 wt% or more, further preferably 1 wt% or more, further more preferably 2 wt% or more, 3 wt% or more, 4 wt% or more, or 5 wt% or more, with respect to the cheese analogue.

In the production method of the present invention, the amount of protein to be used is, for example, 50 wt% or less, preferably 40 wt% or less, more preferably 30 wt% or less, further preferably 20 wt% or less, with respect to the cheese analogue.

In the production method of the present invention, the amount of protein to be used is, for example, 0.1 to 50 wt%, preferably 0.2 to 40 wt%, more preferably 0.5 to 30 wt%, further preferably 1 to 20 wt%, further more preferably 2 to 20 wt%, 3 to 20 wt%, 4 to 20 wt%, or 5 to 20 wt%, with respect to the cheese analogue.

In the production method of the present invention, when the amount of use of protein is less than 0.1 wt% with respect to the cheese analogue, the cheese analogue tends to lack nutritional value and oil separation tends to occur during production.

In the production method of the present invention, when the amount of use of protein exceeds 50 wt% with respect to the cheese analogue, an unappetizing cheese analogue with a powdery texture tends to be produced, and gelation tends to occur during production.

In the production method of the present invention, the weight ratio of starch and protein (starch:protein) is, for example, 1:6 to 6:1, preferably, 1:5 to 5:1, more preferably, 1:4 to 4:1, further preferably, 1:3 to 3:1.

In another embodiment, in the production method of the present invention, the weight ratio of starch and protein (starch:protein) is, for example, 1:0.05 to 6, preferably, 1:0.1 to 5, more preferably, 1:0.2 to 4, further preferably, 1:0.3 to 3.

In the production method of the present invention, the weight ratio of starch and fat and/or oil (starch:fat and/or oil) is, for example, 1:0.2 to 12, preferably, 1:0.3 to 11, more preferably, 1:0.4 to 10, further preferably, 1:0.5 to 4.

In the production method of the present invention, the weight ratio of starch, protein, and fat and/or oil (starch:protein:fat and/or oil) is, for example, 1:0.05 to 6:0.2 to 12, preferably, 1:0.1 to 5:0.3 to 11, more preferably, 1:0.2 to 4:0.4 to 10, further preferably, 1:0.3 to 3:0.5 to 4.

In the production method of the present invention, when an endo-type protease is used as the protease, the amount of the endo-type protease to be added is such that the endo-type protease activity is preferably 0.001 to 10000000 U, more preferably 0.01 to 1000000 U, further preferably 0.1 to 100000 U, particularly preferably 1 to 10000 U, with respect to 1 g of the cheese analogue.

In the production method of the present invention, when an exo-type protease is used as the protease, the amount of the exo-type protease to be added is such that the exo-type protease activity is preferably 0.00001 to 1000000 U, more preferably 0.0001 to 100000 U, further preferably 0.001 to 10000 U, particularly preferably 0.01 to 1000 U, with respect to 1 g of the cheese analogue.

In the production method of the present invention, when an endo-type/exo-type protease is used as the protease, the amount of the endo-type/exo-type protease to be added is such that the endo-type protease activity is preferably 0.001 to 10000000 U, more preferably 0.01 to 1000000 U, further preferably 0.1 to 100000 U, particularly preferably 1 to 10000 U, with respect to 1 g of the cheese analogue.

In the production method of the present invention, when an endo-type/exo-type protease is used as the protease, the amount of the endo-type/exo-type protease is such that the exo-type protease activity is preferably 0.00001 to 1000000 U, more preferably 0.0001 to 100000 U, further preferably 0.001 to 10000 U, particularly preferably 0.01 to 1000 U, with respect to 1 g of the cheese analogue.

In the production method of the present invention, when an endo-type/exo-type protease is used as the protease, the amount of the endo-type/exo-type protease is such that the endo-type protease activity is preferably 0.001 to 10000000 U, more preferably 0.01 to 1000000 U, further preferably 0.1 to 100000 U, particularly preferably 1 to 10000 U, with respect to 1 g of the cheese analogue; and the exo-type protease activity is preferably 0.00001 to 1000000 U, more preferably 0.0001 to 100000 U, further preferably 0.001 to 10000 U, particularly preferably 0.01 to 1000 U, with respect to 1 g of the cheese analogue.

In the production method of the present invention, when an endo-type/exo-type protease is used as the protease, the ratio of endo-type protease activity and exo-type protease activity (endo-type protease activity:exo-type protease activity) in the endo-type/exo-type protease is, for example, 1 U:0.0000001 to 10000 U, preferably 1 U:0.000001 to 1000 U, more preferably 1 U:0.00001 to 100 U, further preferably 1 U:0.0001 to 10 U.

In the production method of the present invention, when an endo-type protease and an exo-type protease are used in combination as the protease, the amount of the endo-type protease to be added is such that the enzyme activity is preferably 0.001 to 10000000 U, more preferably 0.01 to 1000000 U, further preferably 0.1 to 100000 U, particularly preferably 1 to 10000 U, with respect to 1 g of the cheese analogue; and the amount of the exo-type protease is such that the enzyme activity is preferably 0.00001 to 1000000 U, more preferably 0.0001 to 100000 U, further preferably 0.001 to 10000 U, particularly preferably 0.01 to 1000 U, with respect to 1 g of the cheese analogue.

In the production method of the present invention, when an endo-type protease and an exo-type protease are used in combination as the protease, the ratio of the amounts of the endo-type protease and the exo-type protease (endo-type protease:exo-type protease) is, for example, 1 U:0.0000001 to 10000 U, preferably 1 U:0.000001 to 1000 U, more preferably 1 U:0.00001 to 100 U, further preferably 1 U:0.0001 to 10 U.

In the production method of the present invention, it is preferable to allow α-glucosidase to act in combination with protease on a mixture containing fat and/or oil, starch, and protein.

In the production method of the present invention, when α-glucosidase is used, the amount of α-glucosidase to be added is such that the enzyme activity is preferably 0.000001 to 10000 U, more preferably 0.00001 to 1000 U, further preferably 0.0001 to 100 U, particularly preferably 0.001 to 10 U, with respect to 1 g of the cheese analogue.

In the production method of the present invention, when an endo-type protease and an α-glucosidase are used, the ratio of the amounts of the endo-type protease and the α-glucosidase (endo-type protease:α-glucosidase) is, for example, 1 U:0.00000001 to 100 U, preferably 1 U:0.0000001 to 10 U, more preferably 1 U:0.000001 to 1 U, further preferably 1 U:0.00001 to 0.1 U.

In the production method of the present invention, when an exo-type protease and an α-glucosidase are used, the ratio of the amounts of the exo-type protease and the α-glucosidase is (exo-type protease:α-glucosidase), for example, 1 U:0.000001 to 10000 U, preferably 1 U:0.00001 to 1000 U, more preferably 1 U:0.0001 to 100 U, further preferably 1 U:0.001 to 10 U.

In the production method of the present invention, when an endo-type/exo-type protease and α-glucosidase are used, the ratio of the amounts of the endo-type/exo-type protease in terms of endo-type protease activity, the endo-type/exo-type protease in terms of exo-type protease activity, and the α-glucosidase (endo-type protease activity:exo-type protease activity:α-glucosidase) is, for example, 1 U:0.00000001 to 100 U:0.000000001 to 10 U, preferably 1 U:0.0000001 to 10 U:0.00000001 to 1 U, more preferably 1 U:0.000001 to 1 U:0.0000001 to 0.1 U, further preferably 1 U:0.00001 to 0.1 U:0.000001 to 0.01 U.

In the production method of the present invention, when a combination of an endo-type protease and an exo-type protease, and α-glucosidase are used, the ratio of the amounts of the endo-type protease, the exo-type protease, and the α-glucosidase (endo-type protease:exo-type protease:α-glucosidase) is, for example, 1 U:0.0000001 to 1000 U:0.00000001 to 100 U, preferably 1 U:0.000001 to 100 U:0.0000001 to 10 U, more preferably 1 U:0.00001 to 10 U:0.000001 to 1 U, further preferably 1 U:0.0001 to 1 U:0.00001 to 0.1 U.

The production method of the present invention preferably includes the following steps :
(i) a step of mixing (emulsifying) fat and/or oil, starch, protein, and optionally-added additives (e.g., seasoning, various amino acids, excipient, flavor, colorant, polysaccharide thickener), and protease (or protease and α-glucosidase) by heating with stirring, while causing the protease (or protease and α-glucosidase) to act on the fat and/or oil, starch, and protein in the mixture (enzyme reaction step),
(ii) a step of further heating with stirring the mixture obtained in step (i) at a temperature at which the starch is gelatinized to obtain a mixture containing gelatinized starch,
(iii) a step of pouring the mixture obtained in step (ii) into a mold, and cooling same to obtain a cheese analogue.

In another embodiment, the following steps may also be included for production by the production method (I) of the present invention.
(i') a step of mixing to emulsify fat and/or oil, starch, protein, and optionally-added additive (e.g., seasoning, various amino acids, excipient, flavor, colorant, polysaccharide thickener), and protease (or protease and α-glucosidase) to obtain an emulsified mixture,
(ii') a step of pouring the emulsified mixture obtained in step (I') into a mold, and then heating same without stirring to cause the protease (or protease and α-glucosidase) to act on the fat and/or oil, starch, and protein in the mixture (enzyme reaction step),
(iii') a step of heating, after completion of the heating in step (ii'), the mixture in the mold without stirring at a temperature at which the starch is gelatinized to obtain a mixture containing the gelatinized starch,
(iv') a step of cooling, after completion of the heating in step (iii'), the mixture in the mold to obtain a cheese analogue.

The stirring in step (i) can be performed by a method known in the food product manufacturing field, and includes, for example, mixing using a blending machine used for manufacturing cheeses, such as food processor, cooker type emulsifier, kettle type emulsifier, vertical high-speed shear emulsifier, scraping heat exchanger, and the like. The stirring temperature in step (i) is, for example, 5 to 60°C. The stirring time of step (i) is, for example, 0.015 to 24 hr.

Stirring in step (ii) is, for example, mixing using the same blending machine as in step (i). Step (i) and step (ii) can be performed consecutively in the same blending machine. The stirring temperature in step (ii) is, for example, 60 to 120°C. The stirring time of step (ii) is, for example, 0.005 to 1 hr.

The mixing (stirring) in step (I') can be performed by a method known in the food product manufacturing field, and includes, for example, mixing using a blending machine used for manufacturing cheeses, such as food processor, cooker type emulsifier, kettle type emulsifier, vertical high-speed shear emulsifier, scraping heat exchanger, and the like. The temperature in the mixing step is, for example, 5 to 60°C. The time of the mixing step can be appropriately selected from the time for emulsifying the mixture.

In the production method of the present invention, the reaction time of protease (or protease and α-glucosidase) with the mixture containing fat and/or oil, starch, and protein (enzyme reaction steps of the above-mentioned step (i), (ii') is not particularly limited as long as the enzyme can act on the substrate substances (fat and/or oil, starch, protein). A preferred practical time is 0.015 to 24 hr. Furthermore, the reaction temperature of protease (or protease and α-glucosidase) (enzyme reaction steps of the above-mentioned steps (i), (ii')) is not particularly limited as long as the enzyme maintains its activity. A practical temperature of action is 5 to 60°C.

The heating temperature in step (iii') is, for example, 60 to 120°C. The heating time of step (iii') is, for example, 0.005 to 1 hr.

The enzyme reaction can be terminated, for example, by heating at 70 to 120°C for 10 to 120 min (enzyme inactivation step). In the production method of the present invention, the enzyme can be inactivated by heating in the above-mentioned steps (ii) and (iii').

In the production method of the present invention, the pH of the mixture containing fat and/or oil, starch, and protein on which protease (or protease and α-glucosidase) acts is, for example, pH 3 to 6.

In the production method of the present invention, the mixture containing fat and/or oil, starch, and protein on which protease (or protease and α-glucosidase) acts contains water.

The amount of water to be used is, for example, 5 to 80% by weight, preferably 15 to 70% by weight, more preferably 25 to 60% by weight, and further preferably 35 to 50% by weight, with respect to the cheese analogue.

In the production method of the present invention, the cheese analogue may contain additives generally used in the field of food products other than the above-mentioned components.

Examples of the additives include flavor (e.g., cheddar flavor (powder, liquid), parmesan flavor (powder, liquid), camembert flavor (powder, liquid), cream cheese flavor (powder, liquid)), seasoning (e.g., salt, yeast extract), colorant, excipient (dextrin, lactose), various amino acid, polysaccharide thickener (e.g., gum arabic, xanthan gum, tamarind seed gum, guargum, locust bean gum, carrageenan, agar) enzymes other than protease and α-glucosidase, and the like.

The amount of the additive to be used is, for example, 1 to 30% by weight with respect to the cheese analogue.

Cheese analogues with improved meltability and/or extensibility when heated can be produced by the above-mentioned production method of the present invention.

In the present invention, "heated" of the "meltability and/or extensibility when heated" refers to, for example, heating to melt and/or extend cheese analogues during cooking or food processing using the cheese analogue as an ingredient. The heating temperature is, for example, 70 to 200°C., and the heating time is, for example, 1 to 20 min. The "when heated" refers to immediately after heating is completed (for example, within 20 min).

In the present invention, the "meltability" refers to the property of the cheese analogue to liquefy, melt, and spread.

In the present invention, the "extensibility" refers to the property of the cheese analogue to stretch like a string.

In the present invention, the "improved meltability when heated" means that the meltability during heating of the cheese analogue produced by adding an enzyme in the present invention is improved as compared with that of a cheese analogue produced without adding an enzyme.

In the present invention, the "improved extensibility when heated" means that the extensibility during heating of the cheese analogue produced by adding an enzyme in the present invention is improved as compared with that of a cheese analogue produced without adding an enzyme.

In the present specification, the effect of improving "meltability and/or extensibility when heated" can be evaluated, for example, by the methods of the below-mentioned Experimental Examples 1, 2, 6, or by a method analogous thereto.

Furthermore, the present invention provides a method for improving the meltability and/or extensibility of cheese analogues when heated, including a step of causing protease (or protease and α-glucosidase) to act on a mixture containing fat and/or oil, starch, and protein during the production of cheese analogues.

In the method for improving the meltability and/or extensibility of cheese analogues when heated of the present invention, the definitions, examples, and amounts of use of fat and/or oil, the definitions, examples, and amounts of use of starches, and the definitions, examples, and amounts of use of proteins, ratios of these components, the definitions, examples, amounts added, and addition methods of enzymes (protease, α-glucosidase), optionally added components, additives, and the like are the same as the definitions, examples, and amounts of use of fat and/or oil, the definitions, examples, and amounts of use of starches, and the definitions, examples, and amounts of use of proteins, ratios of these components, the definitions, examples, amounts added, and addition methods of enzymes (protease, α-glucosidase), optionally added components, additives, and the like explained in the above-mentioned production method of the present invention.

Furthermore, the present invention relates to an enzyme preparation containing protease (or protease and α-glucosidase), for improving the meltability and/or extensibility of cheese analogues when heated, by causing the protease (preferably, protease and α-glucosidase) to act on a mixture containing fat and/or oil, starch, and protein during production of cheese analogues.

In the enzyme preparation of the present invention, the definitions, examples, and amounts of use of fat and/or oil, the definitions, examples, and amounts of use of starches, and the definitions, examples, and amounts of use of proteins, ratios of these components, the definitions, examples, amounts added, and addition methods of enzymes (protease, α-glucosidase), optionally added components, additives, and the like are the same as the definitions, examples, and amounts of use of fat and/or oil, the definitions, examples, and amounts of use of starches, and the definitions, examples, and amounts of use of proteins, ratios of these components, the definitions, examples, amounts added, and addition methods of enzymes (protease, α-glucosidase), optionally added components, additives, and the like explained in the above-mentioned production method of the present invention.

The enzyme preparation of the present invention can improve the meltability and/or extensibility of cheese analogues when heated, when added to and allowed to react with a mixture containing fat and/or oil, starch, and protein, according to the addition method and the amounts to be added of the protease (or protease and α-glucosidase) explained in the above-mentioned production method of the present invention.

The enzyme preparation of the present invention for improving the meltability and/or extensibility of cheese analogues when heated may further contain, in addition to protease (or protease and α-glucosidase), excipients such as dextrin, indigestible dextrin, reduction maltose, and the like, seasoning such as meat extract and the like, protein such as plant protein, gluten, egg white, gelatin, casein, and the like, protein hydrolysate, protein partial hydrolysate, enzyme other than protease and α-glucosidase, emulsifier, chelating agent such as citrate, polyphosphosphate, and the like, reducing agent such as glutathione, cysteine, and the like, other food additives such as alginic acid, alkaline water, fat and/or oil, dye, sour agent, flavor, and the like, and the like. The enzyme preparation of the present invention may be in any form of liquid, paste, granular or powder.

The production method (II) of the present invention characteristically includes
(a) a step of causing protease to act on a mixture containing fat and/or oil, starch, and protein, and
(b) a step of heating with stirring the mixture obtained in step (a).

The definitions, examples, and amounts of use of each component are as described above.

The production method (II) of the present invention preferably includes the following steps:
(i) a step of mixing (emulsifying) fat and/or oil, starch, protein, and optionally-added additives (e.g., seasoning, various amino acids, excipient, flavor, colorant, polysaccharide thickener), and protease by heating with stirring, while causing the protease to act on the fat and/or oil, starch, and protein in the mixture (enzyme reaction step),
(ii) a step of further heating with stirring the mixture obtained in step (i) at a temperature at which the starch is gelatinized to obtain a mixture containing gelatinized starch,
(iii) a step of pouring the mixture obtained in step (ii) into a mold, and cooling same to obtain a cheese analogue.

The stirring in step (i) can be performed by a method known in the food product manufacturing field, and includes, for example, mixing using a blending machine used for manufacturing cheeses, such as food processor, cooker type emulsifier, kettle type emulsifier, vertical high-speed shear emulsifier, scraping heat exchanger, and the like. The stirring temperature in step (i) is, for example, 5 to 60°C. The stirring time of step (i) is, for example, 0.015 to 24 hr.

Stirring in step (ii) is, for example, mixing using the same blending machine as in step (i). Step (i) and step (ii) can be performed consecutively in the same blending machine. The stirring temperature in step (ii) is, for example, 60°C or more (e.g., 60 to 120°C). The stirring time of step (ii) is, for example, 0.005 to 1 hr.

The enzyme reaction can be terminated, for example, by heating at 70 to 120°C for 10 to 120 min (enzyme inactivation step). In the production method of the present invention, the enzyme can be inactivated by heating in the above-mentioned step (ii) .

In the production method (II) of the present invention, the mixture containing fat and/or oil, starch, and protein (mixture of the above-mentioned step (i)) on which protease is caused to act preferably contains polysaccharide thickener (e.g., gum arabic, xanthan gum, tamarind seed gum, guargum, locust bean gum, carrageenan, agar, etc.). Polysaccharide thickener may be used alone or in combination of two or more.

The amount of polysaccharide thickener to be added is, for example, 0.01 to 50% by weight, preferably 0.025 to 40% by weight, more preferably 0.05 to 30% by weight, and further preferably 0.1 to 20% by weight, with respect to the cheese analogue.

In the production method (II) of the present invention, combined use of protease and polysaccharide thickener further suppresses separation of oil as compared with when protease is used alone.

According to the production method (II) of the present invention, gelation of a mixture containing fat and/or oil, starch, and protein and/or separation of oil can be suppressed during production of cheese analogues, by a step of causing protease to act on the mixture and then heating the mixture with stirring.

According to the production method (II) of the present invention, cheese analogues can be produced by an efficient production method of heating a mixture of raw material components with stirring, even when the mixture of raw material components contains a large amount of protein.

In the present specification, the effect of suppressing "gel and/or separation of oil" during production of cheese analogues can be evaluated, for example, by the methods of the below-mentioned Experimental Examples 3 to 5, or by a method analogous thereto.

In addition, the present invention relates to a method for suppressing gelation of a mixture containing fat and/or oil, starch, and protein and/or separation of oil in step (b), including
(a) a step of causing protease to act on the mixture, and
(b) a step of heating with stirring the mixture obtained in step (a), during production of a cheese analogue containing fat and/or oil, starch and protein.

In the method for suppressing the gelation and/or separation of oil of the present invention, the definitions, examples, and amounts of use of fat and/or oil, the definitions, examples, and amounts of use of starches, and the definitions, examples, and amounts of use of proteins, ratios of these components, the definitions, examples, amounts added, and addition methods of protease, optionally added components, additives, and the like are the same as the definitions, examples, and amounts of use of fat and/or oil, the definitions, examples, and amounts of use of starches, and the definitions, examples, and amounts of use of proteins, ratios of these components, the definitions, examples, amounts added, and addition methods of protease, optionally added components, additives, and the like explained in the above-mentioned production method (II) of the present invention.

Furthermore, the present invention relates to an enzyme preparation for suppressing gelation of the aforementioned mixture containing fat and/or oil, starch, and protein and/or separation of oil, which preparation containing protease, for causing the protease to act on the aforementioned mixture, in the above-mentioned method for suppressing gelation and/or separation of oil.

In the enzyme preparation of the present invention, the definitions, examples, and amounts of use of fat and/or oil, the definitions, examples, and amounts of use of starches, and the definitions, examples, and amounts of use of proteins, ratios of these components, the definitions, examples, amounts added, and addition methods of protease, optionally added components, additives, and the like are the same as the definitions, examples, and amounts of use of fat and/or oil, the definitions, examples, and amounts of use of starches, and the definitions, examples, and amounts of use of proteins, ratios of these components, the definitions, examples, amounts added, and addition methods of protease, optionally added components, additives, and the like explained in the above-mentioned production method (II) of the present invention.

The enzyme preparation of the present invention can suppress gelation of a mixture containing fat and/or oil, starch, and protein and/or separation of oil, when added to and allowed to react with the mixture, according to the addition method and the amounts to be added of the protease explained in the above-mentioned production method (II) of the present invention.

The enzyme preparation of the present invention for suppressing gelation of the aforementioned mixture and/or separation of oil may further contain, in addition to protease, excipients such as dextrin, indigestible dextrin, reduction maltose, and the like, seasoning such as meat extract and the like, protein such as plant protein, gluten, egg white, gelatin, casein, and the like, protein hydrolysate, protein partial hydrolysate, enzyme other than protease, emulsifier, chelating agent such as citrate, polyphosphosphate, and the like, reducing agent such as glutathione, cysteine, and the like, other food additives such as alginic acid, alkaline water, fat and/or oil, dye, sour agent, flavor, and the like, and the like. The enzyme preparation of the present invention may be in any form of liquid, paste, granular or powder.

The cheese analogue produced by the production method of the present invention can be used as it is as a food or in combination with other ingredients or general foods.

In the present specification, a food product is a concept that broadly encompasses anything that can be ingested orally (excluding pharmaceutical products), and includes not only so-called "food" but also beverages, health supplements, and foods with health claims (e.g., foods for specified health uses, foods with functional claims, foods with nutritional function), supplements, and the like.

### [Example]

The present invention is explained in more detail in the following by referring to Examples and Experimental Examples, which are not to be construed as limitative.

### [Experimental Example 1] (Evaluation of meltability and extensibility of cheese analogue when heated)

### (Production of cheese analogues of Example 1, Example 1', and Comparative Example 1)

According to the blending ratios shown in Table 1-1, raw materials were emulsified by mixing for 1 min using a food processor (Bamix, manufactured by CHERRY TERRACE Inc.). The obtained mixture was poured into a mold, heated in a constant temperature reservoir at 50°C for 20 min without stirring (enzyme reaction step), successively heated at 95°C for 30 min without stirring (enzyme inactivation step), and cooled in a refrigerator (5°C) for 48 hr to obtain the cheese analogues of Example 1, Example 1', and Comparative Example 1.

**[Table 1-1]**

| raw material | blending ratio (wt%) | | |
|---|---|---|---|
| | Comparative Example 1 | Example 1 | Example 1' |
| waxy corn starch | 11.50 | 11.50 | 11.50 |
| tapioca starch | 11.50 | 11.50 | 11.30 |
| almond protein | 5.00 | 5.00 | 5.00 |
| Proteax (*1) | - | 0.30 | 0.30 |
| α-glucosidase (*2) | - | - | 0.20 |
| gum arabic | 1.80 | 1.80 | 1.80 |
| agar | 1.00 | 1.00 | 1.00 |
| coconut oil | 20.00 | 20.00 | 20.00 |
| water | 44.63 | 44.33 | 44.33 |
| flavor | 1.10 | 1.10 | 1.10 |
| salt | 1.37 | 1.37 | 1.37 |
| various amino acids | 0.78 | 0.78 | 0.78 |
| yeast extract | 1.12 | 1.12 | 1.12 |
| lactic acid | 0.10 | 0.10 | 0.10 |
| colorant | 0.10 | 0.10 | 0.10 |
| total | 100.00 | 100.00 | 100.00 |

| | | | |
|---|---|---|---|
| *1: Proteax (trade name), manufactured by Amano Enzyme Inc., 218000 U/g (endo-type activity) 1,400 U/g (exo-type activity), (endo-type/exo-type protease) The amount "0.30" of protease (Proteax) in the cheese analogues of Example 1 and Example 1' corresponds to endo-type activity 654 U and exo-type activity 4.2 U when converted to enzyme activity per 1 g of cheese analogue. *2: α-glucosidase "Amano" (trade name), manufactured by Amano Enzyme Inc., 120 U/g The amount "0.20" of α-glucosidase in the cheese analogue of Example 1' corresponds to 0.24 U when converted to enzyme activity per 1 g of cheese analogue. | | | |

### (Evaluation of meltability and extensibility of cheese analogue when heated)

The meltability and extensibility of cheese analogue when heated of the cheese analogues of Example 1, Example 1', and Comparative Example 1 obtained above were evaluated by the method shown below.

The cheese analogues of Example 1, Example 1', and Comparative Example 1 (weight: 3 g, size: 15 mm×30 mm×5 mm) were heated in an oven at 180°C for 10 min.

After completion of the heating, the cheese analogues of Example 1, Example 1', and Comparative Example 1 were visually observed to evaluate the meltability. Photographs of the cheese analogues of Example 1, Example 1', and Comparative Example 1 before heating and after heating are shown in Fig. 1 (photograph on the left of arrow: before heating, photograph on the right of arrow: after heating).

Immediately after completion of the heating, the cheese analogues of Example 1, Example 1', and Comparative Example 1 were pinched with chopsticks and stretched upward, and the maximum length of the cheese analogues that did not break was observed to evaluate the extensibility. Photographs taken when the cheese analogues of Examples 1 and Example 1' were stretched to the maximum length that did not break are shown in Fig. 2. The cheese analogue of Comparative Example 1 broke when picked up and could not be stretched at all (Fig. 2).

The meltability and extensibility were evaluated as -, +, ++, +++, ++++ according to the criteria shown in Table 1-2. The results are shown in Table 1-3.

**[Table 1-2]**

| evaluation | meltability | extensibility |
|---|---|---|
| - | no meltability | no extensibility |
| + | has a little meltability | has a little extensibility |
| ++ | has some meltability | has some extensibility |
| +++ | has considerable meltability | has considerable extensibility |
| ++++ | has high meltability | has high extensibility |

**[Table 1-3]**

| | Comparative Example 1 | Example 1 | Example 1' |
|---|---|---|---|
| meltability | - | ++ | +++ |
| extensibility | - | +++ | ++++ |

The results of the meltability evaluation in Fig. 1, Table 1-3 show that all cheese analogues produced by causing protease to act (Example 1, Example 1') were observed to have good meltability as compared with the cheese analogue without addition of enzyme (Comparative Example 1). This indicates that the meltability when heated was improved by causing protease to act.

The results of the extensibility evaluation in Fig. 2, Table 1-3 show that all cheese analogues produced by causing protease to act (Example 1, Example 1') were observed to have good extensibility as compared with the cheese analogue without addition of enzyme (Comparative Example 1). This indicates that the extensibility when heated was improved by causing protease to act.

In addition, by comparison of Example 1 (protease alone) and Example 1' (combined use of protease and α-glucosidase) in the extensibility evaluation in Fig. 2, Table 1-3, it is clear that the extensibility when heated was further improved by the combined use of protease and α-glucosidase, as compared with the use of protease alone.

### [Experimental Example 2] (Evaluation of meltability and/or extensibility of cheese analogue when heated)

### (Production of cheese analogues of Examples 2, 3)

According to the blending ratios shown in Table 2-1, raw materials were emulsified by stirring with heating at 50°C for 20 min (enzyme reaction step) using a heating stirrer (Thermomix (trade name), manufactured by Vorwek). Successively, the mixture was stirred with heating at 100°C for 15 min (enzyme inactivation step). The obtained mixture was filled in a mold and cooled in a refrigerator (5°C) for 48 hr to obtain the cheese analogues of Examples 2, 3.

**[Table 2-1]**

| raw material | blending ratio (wt%) | |
|---|---|---|
| | Example 2 | Example 3 |
| sago starch | 5.90 | - |
| waxy corn starch | 11.60 | 11.50 |
| tapioca starch | - | 11.50 |
| almond protein | 8.00 | 5.00 |
| Proteax (*1) | 0.30 | 0.30 |
| gum arabic | 1.80 | 1.80 |
| agar | 1.00 | 1.00 |
| coconut oil | 23.00 | 20.00 |
| water | 43.83 | 44.33 |
| flavor | 1.10 | 1.10 |
| salt | 1.37 | 1.37 |
| various amino acids | 0.78 | 0.78 |
| yeast extract | 1.12 | 1.12 |
| lactic acid | 0.10 | 0.10 |
| colorant | 0.10 | 0.10 |
| total | 100.00 | 100.00 |

| | | |
|---|---|---|
| *1: Proteax (trade name), manufactured by Amano Enzyme Inc., 218,000 U/g (endo-type activity) 1,400 U/g (exo-type activity), (endo-type/exo-type protease) The amount "0.30" of protease (Proteax) in the cheese analogues of Examples 2, 3 corresponds to endo-type activity 654 U and exo-type activity 4.2 U when converted to enzyme activity per 1 g of cheese analogue. | | |

### (Evaluation of meltability and extensibility of cheese analogue when heated)

The meltability and extensibility cheese analogue when heated of the cheese analogues of Examples 2, 3 obtained above were evaluated by the method shown below.

The cheese analogues of Examples 2, 3 (weight: 3 g, size: 15 mm×30 mm×5 mm) were heated in an oven at 180°C for 10 min.

After completion of the heating, the cheese analogues of Examples 2, 3 were visually observed to evaluate the meltability. Photographs of the cheese analogues of Examples 2, 3 before heating and after heating are shown in Fig. 3 (photograph on the left of arrow: before heating, photograph on the right of arrow: after heating).

Immediately after completion of the heating, the cheese analogues of Examples 2, 3 were pinched with chopsticks and stretched upward, and the maximum length of the cheese analogues that did not break was observed to evaluate the extensibility. Photographs taken when the cheese analogues of Examples 2, 3 were stretched to the maximum length that did not break are shown in Fig. 4.

The meltability and extensibility were evaluated as -, +, ++, +++, ++++ according to the criteria shown in Table 2-2. The results are shown in Table 2-3.

**[Table 2-2]**

| evaluation | meltability | extensibility |
|---|---|---|
| - | no meltability | no extensibility |
| + | has a little meltability | has a little extensibility |
| ++ | has some meltability | has some extensibility |
| +++ | has considerable meltability | has considerable extensibility |
| ++++ | has high meltability | has high extensibility |

**[Table 2-3]**

| | Example 2 | Example 3 |
|---|---|---|
| meltability | ++ | ++ |
| extensibility | ++ | +++ |

The results of the meltability evaluation in Fig. 3, Table 2-3 show that the cheese analogues produced by causing protease to act (Examples 2, 3) were observed to have good meltability. This indicates that the meltability when heated was improved by causing protease to act.

The results of extensibility evaluation in Fig. 4, Table 2-3 show that the cheese analogues produced by causing protease to act (Examples 2, 3) were observed to have good extensibility. This indicates that the extensibility when heated was improved by causing protease to act.

### [Experimental Example 3] Study on improving production suitability (suppressive effect on gelation and separation of oil) by adding protease

### (Production of cheese analogues of Example 4, 5)

According to the blending ratios shown in Table 3-1, raw materials were stirred with heating at 50°C for 20 min (enzyme reaction step) using a heating stirrer (Thermomix (trade name), manufactured by Vorwerk). Successively, the mixture was stirred with heating at 100°C for 15 min (enzyme inactivation step). Photographs of the mixture after stirring with heating at 100°C for 15 min are shown as the middle photograph (mixture of Example 4) and right photograph (mixture of Example 5) in Fig. 5.

The obtained mixture was filled in a mold and cooled in a refrigerator (5°C) for 48 hr to obtain the cheese analogues of Examples 4, 5.

### (Production of cheese analogue of Comparative Example 2)

According to the blending ratios shown in Table 3-1, raw materials were stirred with heating at 50°C for 20 min using a heating stirrer (Thermomix (trade name), manufactured by Vorwerk). Successively, the mixture was stirred with heating at 100°C for 15 min. Photograph of the mixture after stirring with heating at 100°C for 15 min is shown as the left photograph (mixture of Comparative Example 2) in Fig. 5.

Oil separation and gel collapse occurred in the obtained mixture, and thus a cheese analogue could not be produced.

**[Table 3-1]**

| raw material | blending ratio (wt%) | | |
|---|---|---|---|
| | Comparative Example 2 | Example 4 | Example 5 |
| sago starch | 5.90 | 5.90 | 5.90 |
| waxy corn starch | 11.60 | 11.60 | 11.60 |
| almond protein | 8.00 | 8.00 | 8.00 |
| Proteax (*1) | - | 0.30 | 0.30 |
| gum arabic | - | - | 1.80 |
| agar | 1.00 | 1.00 | 1.00 |
| coconut oil | 24.60 | 24.60 | 24.60 |
| water | 44.33 | 44.03 | 42.23 |
| flavor | 1.10 | 1.10 | 1.10 |
| salt | 1.37 | 1.37 | 1.37 |
| various amino acids | 0.78 | 0.78 | 0.78 |
| yeast extract | 1.12 | 1.12 | 1.12 |
| lactic acid | 0.10 | 0.10 | 0.10 |
| colorant | 0.10 | 0.10 | 0.10 |
| total | 100.00 | 100.00 | 100.00 |

| | | | |
|---|---|---|---|
| *1: Proteax (trade name), manufactured by Amano Enzyme Inc., 218,000 U/g endo-type activity) 1,400 U/g (exo-type activity), (endo-type/exo-type protease) | | | |

The amount "0.30" of protease (Proteax) in the cheese analogues of Examples 4, 5 corresponds to endo-type activity 654 U and exo-type activity 4.2 U when converted to enzyme activity per 1 g of cheese analogue.

The presence or absence of suppression of oil separation and suppression of gel collapse during production of cheese analogues of Examples 4, 5, Comparative Example 2 was evaluated as -, +, ++, +++, ++++ according to the criteria shown in Table 3-2. The results are shown in Table 3-3.

**[Table 3-2]**

| evaluation | suppression of oil separation | suppression of gel collapse |
|---|---|---|
| - | oil is separated | gel collapses |
| + | oil is a little separated | gel collapse is a little suppressed |
| ++ | oil separation is suppressed somewhat | gel collapse is suppressed somewhat |
| +++ | oil separation is considerably suppressed | gel collapse is considerably suppressed |
| ++++ | oil separation is highly suppressed | gel collapse is highly suppressed |

**[Table 3-3]**

| | Comparative Example 2 | Example 4 | Example 5 |
|---|---|---|---|
| suppression of oil | - | +++ | ++++ |
| suppression of gel collapse | - | ++++ | ++++ |

From the results in Fig. 5, Table 3-3, it was shown that gelation of a mixture of raw material components (mixture containing fat and/or oil, starch, and protein) (gel collapse) and separation of oil were developed in a step of heating the mixture with stirring at 100°C in Comparative Example 2 in which protease was not added.

In addition, from the results in Fig. 5, Table 3-3, it was shown that gelation of a mixture of raw material components (mixture containing fat and/or oil, starch, and protein) (gel collapse) and separation of oil were suppressed by a step of causing protease to act on the mixture and then stirring the mixture with heating in Examples 4, 5.

That is, the production suitability was improved in Examples 4, 5 as compared with Comparative Example 2.

In addition, by comparison of Example 4 and Example 5 in Fig. 5, Table 3-3, it is clear that the separation of oil was further suppressed by the combined use of protease and gum arabic (polysaccharide thickener) as compared with the use of protease alone.

### [Experimental Example 4] Study on improving production suitability (suppressive effect on gelation and separation of oil) by adding protease

### (Production of cheese analogues of Examples 6, 7)

According to the blending ratios shown in Table 4-1, raw materials were stirred with heating at 50°C for 20 min (enzyme reaction step) using a heating stirrer (Thermomix (trade name), manufactured by Vorwerk). Successively, the mixture was stirred with heating at 100°C for 15 min (enzyme inactivation step). Photographs of the mixture after stirring with heating at 100°C for 15 min are shown as the left photograph (mixture of Example 6) and right photograph (mixture of Example 7) in Fig. 6.

The obtained mixture was filled in a mold and cooled in a refrigerator (5°C) for 48 hr to obtain the cheese analogues of Examples 6, 7.

**[Table 4-1]**

| raw material | blending ratio (wt%) | |
|---|---|---|
| | Example 6 | Example 7 |
| sago starch | 5.90 | 5.90 |
| waxy corn starch | 11.60 | 11.60 |
| almond protein | 8.00 | 8.00 |
| Protin SD-NY10 (*1) | 0.30 | 0.30 |
| gum arabic | - | 1.80 |
| agar | 1.00 | 1.00 |
| coconut oil | 24.60 | 24.60 |
| water | 44.03 | 42.23 |
| flavor | 1.10 | 1.10 |
| salt | 1.37 | 1.37 |
| various amino acids | 0.78 | 0.78 |
| yeast extract | 1.12 | 1.12 |
| lactic acid | 0.10 | 0.10 |
| colorant | 0.10 | 0.10 |
| total | 100.00 | 100.00 |

| | | |
|---|---|---|
| *1: Protin SD-NY10 (trade name), manufactured by Amano Enzyme Inc., 70,000 U/g (endo-type protease), The amount "0.30" of protease (Protin SD-NY10) in the cheese analogues of Examples 6-7 corresponds to 210 U when converted to enzyme activity per 1 g of cheese analogue. | | |

The presence or absence of suppression of oil separation and suppression of gel collapse during production of cheese analogues of Examples 6, 7 was evaluated as -, +, ++, +++, ++++ according to the criteria shown in Table 4-2. The results are shown in Table 4-3.

**[Table 4-2]**

| evaluation | suppression of oil separation | suppression of gel collapse |
|---|---|---|
| - | oil is separated | gel collapses |
| + | oil is a little separated | gel collapse is a little suppressed |
| ++ | oil separation is suppressed somewhat | gel collapse is suppressed somewhat |
| +++ | oil separation is considerably suppressed | gel collapse is considerably suppressed |
| ++++ | oil separation is highly suppressed | gel collapse is highly suppressed |

**[Table 4-3]**

| | Example 6 | Example 7 |
|---|---|---|
| suppression of oil separation | ++ | ++++ |
| suppression of gel collapse | +++ | ++++ |

From the results in Fig. 6, Table 4-3, it was shown that gelation of a mixture of raw material components (mixture containing fat and/or oil, starch, and protein) (gel collapse) and separation of oil were suppressed (production suitability was improved) by a step of causing protease to act on the mixture and then stirring the mixture with heating in Examples 6, 7.

In addition, by comparison of Example 6 and Example 7 in Fig. 6, Table 4-3, it is clear that the separation of oil was further suppressed by the combined use of protease and gum arabic (polysaccharide thickener) as compared with the use of protease alone.

### [Experimental Example 5] Study on improving production suitability (suppressive effect on gelation and separation of oil) by adding exo-type protease

### (Production of cheese analogue of Example 8)

According to the blending ratios shown in Table 5-1, raw materials were stirred with heating at 50°C for 20 min (enzyme reaction step) using a heating stirrer (Thermomix (trade name), manufactured by Vorwerk). Successively, the mixture was stirred with heating at 100°C for 15 min (enzyme inactivation step). Photograph of the mixture after stirring with heating at 100°C for 15 min is shown as the right photograph (mixture of Comparative Example 8) in Fig. 7.

The obtained mixture was filled in a mold and cooled in a refrigerator (5°C) for 48 hr to obtain the cheese analogues of Example 8.

### (Production of cheese analogue of Comparative Example 3)

According to the blending ratios shown in Table 5-1, raw materials were stirred with heating at 50°C for 20 min using a heating stirrer (Thermomix (trade name), manufactured by Vorwerk). Successively, the mixture was stirred with heating at 100°C for 15 min. Photograph of the mixture after stirring with heating at 100°C for 15 min is shown as the left photograph (mixture of Comparative Example 3) in Fig. 7.

Oil separation and gel collapse occurred in the obtained mixture, and thus a cheese analogue could not be produced.

**[Table 5-1]**

| raw material | blending ratio (wt%) | | |
|---|---|---|---|
| | Comparative Example 3 | | Example 8 |
| waxy corn starch | 11.50 | | 11.50 |
| tapioca starch | 11.50 | | 11.50 |
| almond protein | 5.00 | | 5.00 |
| aminopeptidase (*1) | - | | 0.30 |
| gum arabic | 1.80 | | 1.80 |
| agar | 1.00 | | 1.00 |
| coconut oil | 20.00 | | 20.00 |
| water | 44.63 | | 44.33 |
| flavor | 1.10 | | 1.10 |
| salt | 1.37 | | 1.37 |
| various amino acids | 0.78 | | 0.78 |
| yeast extract | 1.12 | | 1.12 |
| lactic acid | 0.10 | | 0.10 |
| colorant | 0.10 | | 0.10 |
| total | 100.00 | | 100.00 |

| | | | |
|---|---|---|---|
| *1: aminopeptidase Denateam LEP 10P (trade name), manufactured by Nagase ChemteX Corporation, 900 U/g (exo-type activity) (exo-type protease) | | | |

The amount of exo-type protease in the cheese analogue of test 2 corresponds to exo-type activity 2.7 U when converted to enzyme activity per 1 g of cheese analogue.

The presence or absence of suppression of oil separation and suppression of gel collapse during production of cheese analogues of Example 8, Comparative Example 3 was evaluated as -, +, ++, +++, ++++ according to the criteria shown in Table 5-2. The results are shown in Table 5-3.

**[Table 5-2]**

| evaluation | suppression of oil separation | suppression of gel collapse |
|---|---|---|
| - | oil is separated | gel collapses |
| + | oil is a little separated | gel collapse is a little suppressed |
| ++ | oil separation is suppressed somewhat | gel collapse is suppressed somewhat |
| +++ | oil separation is considerably suppressed | gel collapse is considerably suppressed |
| ++++ | oil separation is highly suppressed | gel collapse is highly suppressed |

**[Table 5-3]**

| | Comparative Example 3 | Example 8 |
|---|---|---|
| suppression of oil separation | - | ++ |
| suppression of gel collapse | - | ++ |

From the results in Fig. 7, Table 5-3, it was shown that gelation of a mixture of raw material components (mixture containing fat and/or oil, starch, and protein) (gel collapse) and separation of oil were developed in a step of heating the mixture with stirring at 100°C in Comparative Example 2 in which exo-type protease was not added.

In addition, from the results in Fig. 7, Table 5-3, it was shown that gelation of a mixture of raw material components (mixture containing fat and/or oil, starch, and protein) (gel collapse) and separation of oil were suppressed by a step of causing exo-type protease to act on the mixture and then stirring the mixture with heating in Example 8.

That is, the production suitability was improved in Example 8 as compared with Comparative Example 3.

### [Experimental Example 6] (Evaluation of meltability and extensibility of cheese analogue when heated)

### (Production of cheese analogues of Examples 9 - 12 and Comparative Example 4)

According to the blending ratios shown in Table 6-1, raw materials were emulsified by mixing for 1 min using a food processor (Bamix, manufactured by CHERRY TERRACE Inc.). The obtained mixture was poured into a mold, heated in a constant temperature reservoir at 50°C for 20 min without stirring (enzyme reaction step), successively heated at 95°C for 30 min without stirring (enzyme inactivation step), and cooled in a refrigerator (5°C) for 48 hr to obtain the cheese analogues of Examples 9 - 12 and Comparative Example 4.

**[Table 6-1]**

| raw material | blending ratio (wt%) | | | | |
|---|---|---|---|---|---|
| | Comparative Example 4 | Example 9 | Example 10 | Example 11 | Example 12 |
| waxy corn starch | 11.50 | 11.50 | 11.50 | 11.50 | 11.50 |
| tapioca starch | 11.50 | 11.50 | 11.50 | 11.50 | 11.50 |
| almond protein | 6.00 | 6.00 | 6.00 | 6.00 | 6.00 |
| Proteax (*1) | - | 0.30 | - | - | - |
| Protin SD-NY10 (*2) | - | - | 0.30 | - | 0.30 |
| aminopeptidase (*3) | - | - | - | 0.30 | 0.30 |
| gum arabic | 1.83 | 1.83 | 1.83 | 1.83 | 1.83 |
| coconut oil | 20.00 | 20.00 | 20.00 | 20.00 | 20.00 |
| water | 45.18 | 44.88 | 44.88 | 44.88 | 44.58 |
| flavor | 1.10 | 1.10 | 1.10 | 1.10 | 1.10 |
| salt | 1.37 | 1.37 | 1.37 | 1.37 | 1.37 |
| various amino acids | 0.12 | 0.12 | 0.12 | 0.12 | 0.12 |
| yeast extract | 1.00 | 1.00 | 1.00 | 1.00 | 1.00 |
| lactic acid | 0.30 | 0.30 | 0.30 | 0.30 | 0.30 |
| colorant | 0.10 | 0.10 | 0.10 | 0.10 | 0.10 |
| total | 100.0 | 100.0 | 100.0 | 100.0 | 100.0 |

| | | | | | |
|---|---|---|---|---|---|
| *1: Proteax (trade name), manufactured by Amano Enzyme Inc., 218,000 U/g (endo-type activity), 1,400 U/g (exo-type activity) (endo-type/exo-type protease) The amount "0.30" of endo-type/exo-type protease in the cheese analogues of Example 9 corresponds to endo-type activity 654 U and exo-type activity 4.2 U when converted to enzyme activity per 1 g of cheese analogue. *2: Protin SD-NY10 (trade name), manufactured by Amano Enzyme Inc. 70,000 U/g (endo-type activity), (endo-type protease) The amount "0.30" of endo-type protease in the cheese analogue of Example 10, 12 corresponds to endo-type activity 210 U when converted to enzyme activity per 1 g of cheese analogue. *3: aminopeptidase Denateam LEP 10P (trade name), manufactured by Nagase ChemteX Corporation, 900 U/g (exo-type activity) (exo-type protease) | | | | | |

The amount "0.30" of exo-type protease in the cheese analogues of Examples 11, 12 corresponds to exo-type activity 2.7 U when converted to enzyme activity per 1 g of cheese analogue.

### (Evaluation of meltability and extensibility of cheese analogue when heated)

The meltability and extensibility cheese analogue when heated of the cheese analogues of Examples 9-12 and Comparative Example 4 obtained above were evaluated by the method shown below.

The cheese analogues of Examples 9-12 and Comparative Example 4 (weight: 3 g, size: 15 mm×30 mm×5 mm) were heated in an oven at 180°C for 10 min.

After completion of the heating, the cheese analogues of Examples 9-12 and Comparative Example 4 were visually observed to evaluate the meltability. Photographs of the cheese analogues of Examples 9-12 and Comparative Example 4 before heating and after heating are shown in Fig. 8 (photograph at the upper side of arrow: before heating, photograph on the lower side of arrow: after heating).

Immediately after completion of the heating, the cheese analogues of Examples 9-12 and Comparative Example 4 were pinched with chopsticks and stretched upward, and the maximum length of the cheese analogues that did not break was observed to evaluate the extensibility. Photographs taken when the cheese analogues of Examples 9-12 were stretched to the maximum length that did not break are shown in Fig. 9. The cheese analogue of Comparative Example 4 broke when picked up and could not be stretched at all (Fig. 9).

The meltability and extensibility were evaluated as -, +, ++, +++, ++++ according to the criteria shown in Table 6-2. The results are shown in Table 6-3.

**[Table 6-2]**

| evaluation | meltability | extensibility |
|---|---|---|
| - | no meltability | no extensibility |
| + | has a little meltability | has a little extensibility |
| ++ | has some meltability | has some extensibility |
| +++ | has considerable meltability | has considerable extensibility |
| ++++ | has high meltability | has high extensibility |

**[Table 6-3]**

| | Comparative Example 4 | Example 9 | Example 10 | Example 11 | Example 12 |
|---|---|---|---|---|---|
| meltability | - | +++ | +++ | ++ | +++ |
| extensibility | - | +++ | +++ | ++ | +++ |

The results of the meltability evaluation in Fig. 8, Table 6-3 show that the cheese analogue produced by causing endo-type/exo-type protease to act (Example 9), the cheese analogue produced by causing endo-type protease to act (Example 10), the cheese analogue produced by causing exo-type protease to act (Example 11), and the cheese analogue produced by causing endo-type protease and exo-type protease in combination to act (Example 12) were observed to have good meltability as compared with the cheese analogue without addition of enzyme (Comparative Example 4). This indicates that the meltability when heated was improved by causing protease to act.

The results of the extensibility evaluation in Fig. 9, Table 6-3 show that the cheese analogue produced by causing endo-type/exo-type protease to act (Example 9), the cheese analogue produced by causing endo-type protease to act (Example 10), the cheese analogue produced by causing exo-type protease to act (Example 11), and the cheese analogue produced by causing endo-type protease and exo-type protease in combination to act (Example 12) were observed to have good extensibility as compared with the cheese analogue without addition of enzyme (Comparative Example 4). This indicates that the extensibility when heated was improved by causing protease to act.

### [Industrial Applicability]

According to the present invention, cheese analogues with improved meltability and/or extensibility when heated can be produced by causing protease to act on a mixture containing fat and/or oil, starch, and protein.

According to the present invention, gelation of a mixture containing fat and/or oil, starch, and protein and/or separation of oil in a step of heating the mixture with stirring can be suppressed by a step of causing protease to act on the mixture during production of cheese analogues.

This application is based on patent application No. 2021-144901 filed in Japan, the contents of which are encompassed in full herein.

## Claims

1. A method for producing a cheese analogue, comprising a step of causing protease to act on a mixture comprising fat and/or oil, starch, and protein.

2. The production method according to claim 1, wherein the protease is selected from the group consisting of
(1) an endo-type/exo-type protease,
(2) a combination of an endo-type protease and an exo-type protease,
(3) an exo-type protease, and
(4) an endo-type protease.

3. The production method according to claim 1 or 2, further comprising causing α-glucosidase to act on the mixture comprising fat and/or oil, starch, and protein.

4. A method for improving meltability and/or extensibility of a cheese analogue when heated, comprising a step of causing protease to act on a mixture comprising fat and/or oil, starch, and protein during production of the cheese analogue.

5. The method for improving meltability and/or extensibility according to claim 4, wherein the protease is selected from the group consisting of
(1) an endo-type/exo-type protease,
(2) a combination of an endo-type protease and an exo-type protease,
(3) an exo-type protease, and
(4) an endo-type protease.

6. The method for improving meltability and/or extensibility according to claim 4 or 5, further comprising causing α-glucosidase to act on the mixture comprising fat and/or oil, starch, and protein.

7. An enzyme preparation for improving meltability and/or extensibility of a cheese analogue when heated, which preparation comprising protease, wherein the preparation is for causing the protease to act on a mixture comprising fat and/or oil, starch, and protein during production of the cheese analogue.

8. The enzyme preparation according to claim 7, wherein the protease is selected from the group consisting of
(1) an endo-type/exo-type protease,
(2) a combination of an endo-type protease and an exo-type protease,
(3) an exo-type protease, and
(4) an endo-type protease.

9. The enzyme preparation according to claim 7 or 8, comprising the protease and α-glucosidase for causing the α-glucosidase to act on the mixture comprising fat and/or oil, starch, and protein.

10. A method for producing a cheese analogue comprising fat and/or oil, starch and protein, comprising
(a) a step of causing protease to act on a mixture comprising fat and/or oil, starch, and protein, and
(b) a step of heating with stirring the mixture obtained in step (a).

11. The production method according to claim 10, wherein the protease is selected from the group consisting of
(1) an endo-type/exo-type protease,
(2) a combination of an endo-type protease and an exo-type protease,
(3) an exo-type protease, and
(4) an endo-type protease.

12. The production method according to claim 10 or 11, wherein the mixture further comprises a polysaccharide thickener.

13. A method for suppressing gelation of a mixture comprising fat and/or oil, starch, and protein and/or separation of oil in step (b), comprising
(a) a step of causing protease to act on the mixture, and
(b) a step of heating with stirring the mixture obtained in step (a), during production of a cheese analogue comprising fat and/or oil, starch and protein.

14. The suppressing method according to claim 13, wherein the protease is selected from the group consisting of
(1) an endo-type/exo-type protease,
(2) a combination of an endo-type protease and an exo-type protease,
(3) an exo-type protease, and
(4) an endo-type protease.

15. The suppression method according to claim 13 or 14, wherein the mixture further comprises a polysaccharide thickener.

16. An enzyme preparation for suppressing gelation of the mixture comprising fat and/or oil, starch, and protein and/or separation of oil, which preparation comprising protease, for causing the protease to act on the mixture, in the method according to any one of claims 13 to 15.
